# EUROPEAN PATENT APPLICATION

(11) **EP 2 893 897 A1**
(43) Date of publication of application: **15.07.2015**
(21) Application number: 15150683.9
(22) Date of filing: 09.01.2015
(51) Int. Cl.: A61B 18/14, A61B 17/12, A61M 25/01

(54) **Energy-induced embolization system**

(30) Priority: 10.01.2014 US 201461925944 P
(71) Applicant: Cook Medical Technologies LLC, Bloomington, IN 47404 (US)
(72) Inventor: Guimaraes, Marcelo Silveira, Mount Pleasant, South Carolina 29464 (US)
(74) Representative: Garratt, Peter Douglas

(57) **Abstract**

An energy-induced embolization system comprises an introducer sheath; a first catheter disposed through the longitudinal opening of the introducer sheath; a conduit having a first end disposed in fluid communication with an inside portion of the introducer sheath and having a second end disposed in fluid communication with an inside portion of the first catheter, wherein antegrade blood flow is establishable in a lumen of a target vessel through a path between the inside portion of the introducer sheath, through an inside portion of the conduit, through the inside portion of the first catheter, and exiting through the distal portion of the first catheter; and an energy delivery wire. A monopolar or bipolar energy delivery system delivers radiofrequency energy to the energy delivery wire.

## Description

### FIELD OF THE INVENTION

The present invention relates generally to embolization techniques in the field of interventional radiology and specifically to an endovascular system for electrocoagulation in small to medium size blood vessels, arteries or veins.

### BACKGROUND OF THE INVENTION

Embolotherapy has become an indispensable part of interventional radiology procedures and of medicine in general. Several diseases and post medical procedure complications are currently treated with minimally invasive embolization techniques. Typically, a skilled interventionist with experience in coaxial microcatheterization techniques is required. Through an endovascular approach, the diagnostic catheter or microcatheter is advanced to the embolization site. Following a pre-embolization angiogram, embolic agents such as gel foam, microparticles (e.g. polyvinyl alcohol), steel and platinum coils, vascular plugs or liquid agents like cyanoacrylate and Onyx are used in the target vessel. The selection of the embolic agent depends on several variables, including how quick hemostasis is desired, blood vessel flow velocity and diameter, patient coagulation status, desire to have temporary or permanent vessel occlusion, risk of non-target embolization and stability of the delivery catheter. The non-target embolization (reflux or distal migration) of an embolic material is always a risk, and sometimes it cannot be easily avoided, requiring a highly skilled interventionist. At this point, none of these embolic agents have proven to be both user friendly and ideal for most embolization procedures or when distal, controlled, superselective and rapid occlusion of small vessels is desired.

There has been an expansion in the indications for embolization procedures and some have become more complex, requiring well trained interventionists, ideally having experience with several different embolic agents. In order to provide a rapid and effective method of embolization, attention has been turned to electrocoagulation. Heat is produced when sufficient electricity is passed through biological tissue. When this heat reaches a critical temperature, proteins denature and form a material called coagulum. The phenomenon of coagulum formation caused by electrical energy is termed electrocoagulation. It has also been observed that alternating current of greater than 10,000 cycles per second (i.e., 10 kHz) at moderate levels did not produce significant pain or generalized muscle contraction in the human body. Alternating current above this cycling rate is known as radiofrequency (RF). By applying RF energy to an area just proximal to the aperture of a bleeding vessel, surgeons have been able to use electrocoagulation to achieve hemostasis and vessel occlusion quickly, safely, and effectively using external monopolar or bipolar cautery systems. The heat energy generated from RF current causes vessel occlusion by the following mechanisms: coagulation of blood proteins resulting in the formation of an occlusive coagulum and induction of thrombosis following vessel wall injuries.

The use of endovascular delivery of energy for occlusion of vessels is known. There are many devices in the market that deliver energy (RF, microwave, or laser) that is used for venous ablation in the lower extremities such as for example patients with varicose veins. However, in the arterial system where the blood flow is faster, the energy delivery for embolization may be inefficient due to the "heat-sink effect" (i.e., the heat generated by the energy-inducing probe is taken away by the blood flow faster than it can be generated for local effect).

Accordingly, there is a need for a system that provides for blood flow control in combination with the focal delivery of energy for electrocoagulation so that the system can be used in any small and mid-sized blood vessel, artery or vein.

### SUMMARY OF THE INVENTION

The present invention meets the above-described need by providing an energy-induced embolization system. The system includes an introducer sheath having an inner wall defining a longitudinal opening extending from a proximal portion to a distal portion of the introducer sheath; a first catheter having an outer wall and having a longitudinal opening extending from a proximal portion to a distal portion of the first catheter, the first catheter disposed through the longitudinal opening of the introducer sheath; a conduit having a first end disposed in fluid communication with an inside portion of the introducer sheath and having a second end disposed in fluid communication with an inside portion of the first catheter, wherein antegrade blood flow is establishable in a lumen of a target vessel through a path between the inside portion of the introducer sheath, through an inside portion of the conduit, through the inside portion of the first catheter, and exiting through the distal portion of the first catheter (for example taking the form of a reperfusion catheter as disclosed in U.S. Patent Publication No. US 2011/0301571 entitled "Reperfusion Catheter System"; and an energy delivery wire. The wire may be 0.035 inch diameter and may be used through 4-5 Fr diagnostic catheters or the wire can be 0.018 inch diameter that can be used with any 0.018 inch lumen compatible microcatheter. The energy delivery system may be monopolar or bipolar. The monopolar system may have a ground pad that is attached to the patient's thigh in order to close the energy circuit to avoid skin burns. The energy delivery wire may be provided with a detachable probe and there are many possible tip configurations. For example, in the monopolar version the tips may be straight or helical. The bipolar system maybe provided with a straight wire and a "V" shape tip configuration. The "V" shape tip is exposed after retraction of the insulator polymer. The tip of the bipolar system may also be provided in straight and helical configurations.

The system provides for embolization with no hardware or embolic agent left behind (the active energy-inducing tip may be detached if necessary) so that there is no artifact in future control image studies (ultrasound, magnetic resonance, computer tomography and the like). Also, there is no non-target embolization, and the delivery of the energy is not affected by the "heat-sink effect". The system does not require microcatheterization of the bleeding site itself in order to deliver the embolic agent like in all other embolization techniques. It is a simpler technique, as it only requires the segment of the wire that delivers the energy to be inside the target blood vessel.

The system provides a significant advantage in embolization procedures of high-flow blood vessels where there is a risk of non-target embolization or of unsuccessful embolization due to fast blood flow - as it prevents backflow from the embolization site and allows interruption and reperfusion with antegrade flow when desired. Also, in hypocoagulable state patients (difficult to form clots), blood vessel occlusion (embolization) may be achieved as the system causes desiccation of the vessel wall leading to shrinkage and retraction of the vessel.

Unlike with surgical electrocoagulation, in which the surgeon can see the vessel from the outside, the result of the intra-luminal endovascular coagulation is difficult to evaluate. However, the system provides easy to control angiograms through the reperfusion catheter, and the embolization procedure endpoint can be easily determined which adds safety and objectivity to the procedure.

The energy-induced embolization system for embolotherapy of the present invention may be used in mid-sized and small blood vessels (regardless of the velocity in the blood vessel), with the possibility to deploy or detach the probe (segment of the device that delivers energy) as needed. The system is available in regular and low profiles and in monopolar and bipolar systems.

### BRIEF DESCRIPTION OF THE DRAWINGS

The invention is illustrated in the drawings in which like reference characters designate the same or similar parts throughout the figures of which:
Fig. 1 is a schematic view of the system of the present invention having a microcatheter and a helical tip on the probe;
Fig. 2 is a schematic view of an alternate embodiment with an inflatable balloon disposed on the microcatheter;
Fig. 3 is a schematic view of another alternate embodiment of the system having a microcatheter and a straight tip on the probe;
Fig. 4 is a schematic view of an alternate embodiment with a straight tip on the probe and having a microcatheter with an inflatable balloon;
Fig. 5 is a schematic view of an alternate embodiment with a wire having a helical probe deployed directly through a balloon catheter; and
Fig. 6 is a schematic view of an alternate embodiment with a wire having a straight probe tip deployed directly through the balloon catheter.

### DETAILED DESCRIPTION OF THE INVENTION

Referring to the figures generally, an example of the energy-induced embolization system may be designed for 0.018" and 0.035" platforms, which are typically used in association with a 2-3 French microcatheter (or any 0.018" inner lumen compatible microcatheter) and a 4-5 French diagnostic catheter, respectively. The main components of the system include a reperfusion catheter 10 which is described in U.S. Patent Publication No. 2011/0301571 entitled "Reperfusion Catheter System"; energy delivery wires which may be associated with either a monopolar or bipolar system; and an energy delivery probe that may be detachable and may be configured in different shapes such as, but not limited to, straight, helical or V-shape. The energy inducing embolization system allows absolute blood vessel flow control, either by interrupting it or by creating an artificial antegrade flow. The energy inducing system delivers energy that ultimately blocks (embolizes) the blood vessel by an endovascular approach (intra-luminal cauterization of the blood vessels). The generator adaptor allows control of the power (Watts) and of the energy delivery time (seconds) that the embolization system will apply in the blood vessel. This adaptor may also allow the utilization of monopolar/bipolar RF embolization systems.

In vascular beds with significant distal resistance (in which any flow directed microparticles or liquid embolic agents will be prone to reflux) and in very small and/or tortuous blood vessels that do not allow selective insertion of a microcatheter (e.g. right gastric artery), the 0.018" platform system may be advanced into the blood vessel to be embolized and the procedure may be accomplished without the need of having additional manipulations or attempts at catheterization. In other words, procedure time, radiation exposure and volume of contrast media (contrast-related nephropathy) may be reduced.

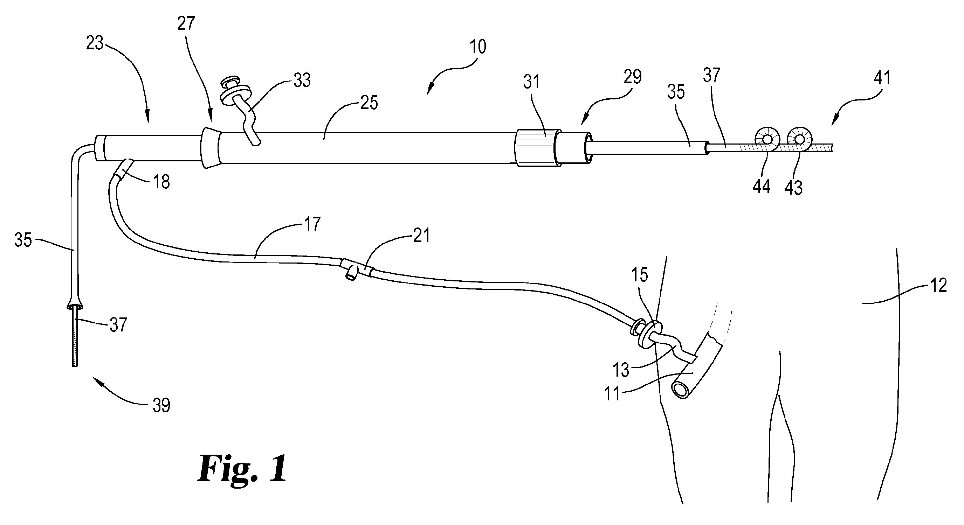

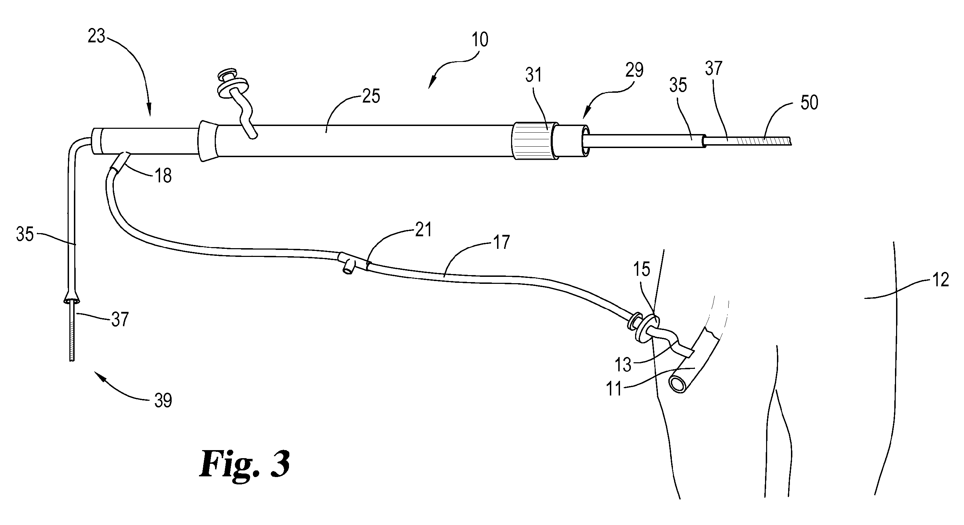

Turning to Fig. 1, the embodiment shown is a 0.018" platform with a microcatheter. For ease of illustration, in each of the figures the reperfusion catheter system is shown outside of the body. In actual use, the balloon catheter is deployed through the center of the introducer sheath 11 shown in the diagram. As shown, the introducer sheath 11 is deployed in the patient's groin area to obtain vascular access (either arterial/venous). A lateral check flow 13 including a stopcock 15 is connected to the hub of the introducer sheath 11. A connecting tube 17 extends between the lateral check flow 13 and a side port 19 in the catheter system. A three-way stopcock 21 may be disposed in the connecting tube 17 to provide access for control angiograms during the procedure. The connecting tube 17 connects the introducer sheath 11 to a side port 18 on a valve system 23. The flow in the connecting tube 17 can be interrupted or created, with reperfusion beyond the occlusion balloon catheter tip, through an artificial antegrade flow at any point during the procedure. The antegrade flow path is described in detail in U.S. Patent Publication No. US 2011/0301571. The detachable valve system 23 (for example, a Touhy-Borst adapter) attaches to a balloon catheter 25. The balloon catheter 25 has a proximal end 27 near the valve 23 and a distal end 29. An inflatable balloon 31 is disposed at the distal end 29. A side port 33 for insufflating the balloon 31 may be located near the proximal end 27. The balloon catheter 25 may be approximately 4-5 French. A microcatheter 35 may be deployed through the center of the balloon catheter 25. An energy inducing wire 37 may be introduced through the center of the microcatheter 35. The wire 37 has a proximal end 39 which connects to a generator (not shown). The wire has a distal end 41 with a helical tip 43. The helical tip 43 may be detached for situations where it is necessary to quickly extract the wire 37 from the lumen of the vessel. The energy-inducing wire 37 has a metal core wire with external insulation. The proximal end 39 which may measure 2 cm in length, has no insulation and may be connected to a generator by a connecting cable. The distally located tip 43, also called a "probe", is not insulated and may measure up to 3 cm long and is the wire segment that delivers energy in a form of radiofrequency, microwave or laser. In the transition between the tip 43 and the energy delivery wire 37 there is a delivery mechanism 44 that allows the tip 43 to be detached if necessary or desired during the procedure. The system shown in Fig. 1 may also be provided with a 0.018" diameter wire 37 having a straight tip 50 (Fig. 3).

In use, once vascular access has been obtained (either arterial/venous), the introducer sheath 11 is introduced into the patient's body 12 to secure the access. Using standard catheterization techniques, the occlusion balloon catheter 25 is advanced selectively up to the target vessel and the lateral check flow 13 of the introducer sheath 11 is connected to tubing 17. A Luer-lock attaches the valve system 23 to the hub of the occlusion balloon catheter 25. At this point, the occlusion balloon catheter compliant balloon 31 is insufflated to stop the blood flow in the target vessel. The introducer sheath lateral check flow 13 is open and the valve system 23 will allow blood flow coming from the common femoral artery through the reperfusion catheter 10 into the target vessel. This technique provides an artificial antegrade flow in the target vessel, which is important to keep the distal perfusion beyond the inflated occlusion balloon 31. The blood flow may be controlled, either by opening the valve 21 (creating reperfusion) or closing it (interruption of the flow), and it can also be used for control angiograms after the energy- induced embolization system is activated. The reperfusion catheter 10 may be made of polymers that will provide the specific features of diameter compatibility, flexibility, torque-ability and optimized intraluminal blood flow.

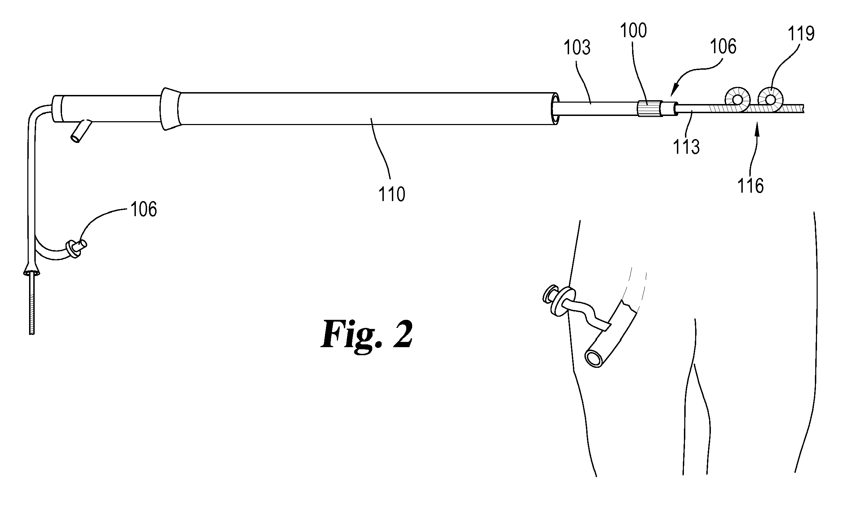

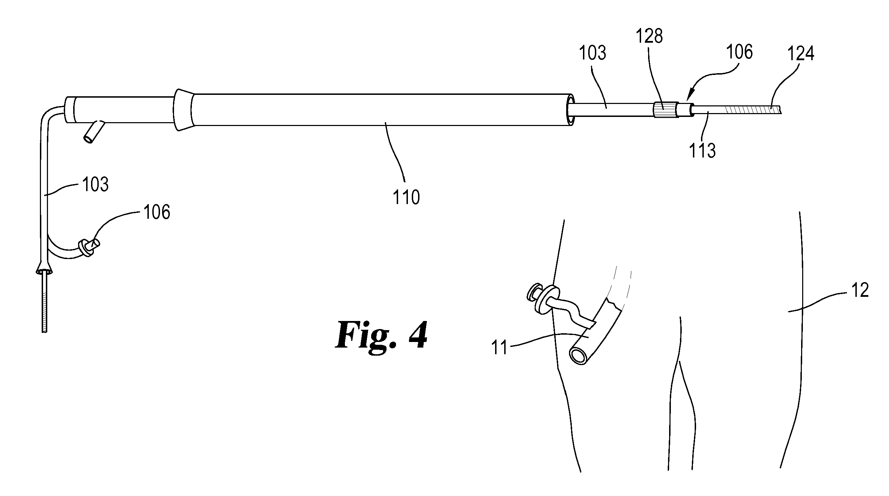

In Fig. 2, an alternate embodiment is shown, in contrast to the system of Fig. 1, an occlusion balloon 100 is disposed on a microcatheter 103. The microcatheter 103 is provided with a lateral port 106 for insufflation of the microcatheter balloon 100. The system includes a diagnostic catheter 110 which may, for example, be 4-5 French. The microcatheter 103 is disposed through the center of the diagnostic catheter 110. The microcatheter 103 has an occlusion balloon 100 at a distal end 106. An energy inducing microwire 113 having a diameter of 0.018" is disposed through the lumen of the microcatheter 103. The distal end 116 of the wire 113 has a helical shape. The helical tip 119 of the wire 113 may be detachable. The system shown in Fig. 2 may also be provided with a 0.018" diameter wire 113 having a straight tip 124 and a microcatheter 103 having an inflatable balloon 128 (Fig. 4).

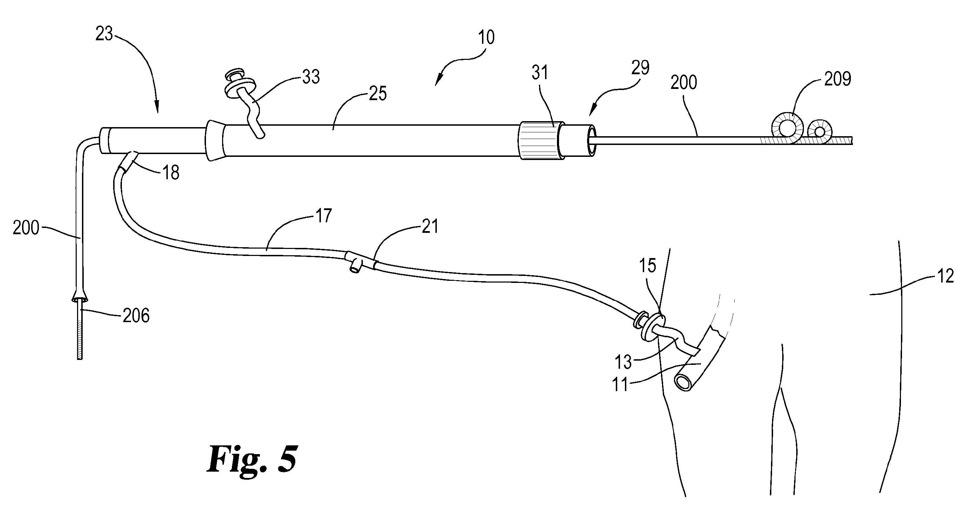

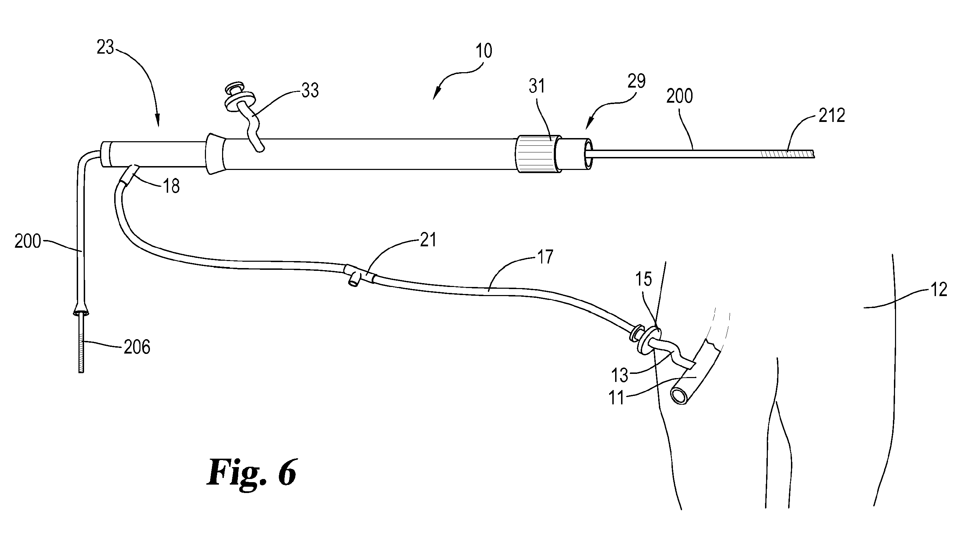

Fig. 5 is an alternate embodiment of the system of the present invention with a 0.035" energy inducing wire 200 that is deployed directly through the balloon catheter 25 without the use of a microcatheter. The wire 200 has a proximal tip 206 that may be uninsulated for connection to a generator (not shown). The wire 200 has a distal tip 209 that is formed in a helical shape and is detachable. The system shown in Fig. 5 may also be provided with a 0.035" wire with a straight tip 212 at the distal end (Fig. 6).

The present invention provides many advantages. The system provides balloon-guided flow control, reperfusion or artificial antegrade flow that may be reestablished at any time during the embolization procedure which is important for control angiograms while the blood vessel spontaneous flow is temporarily blocked by the balloon. Also, the energy delivery device for embolotherapy may be used in mid-sized and small-sized blood vessels (regardless of the velocity in the blood vessel), with the possibility to deploy or detach the probe as needed. The system is available in low and regular profiles (0.018" and 0.035" platforms, respectively) and in monopolar and bipolar systems. The flow control system also prevents distal migration of clots while the probe is creating blood vessel desiccation and clot formation.

In the case of an emergency bleeding secondary to trauma, the balloon of the reperfusion catheter can be inflated and any brisk bleeding quickly controlled. Also, it may prevent sudden death during the embolization procedure, which may take several minutes to be accomplished in challenging anatomical sites. Two mechanisms: (1) a wire with the probe must be advanced distal and proximal to the bleeding site and the energy-inducing embolization system is activated; and (2) proximal balloon inflation controls the bleeding while access with the energy inducing embolization system is obtained. Distal branches with acute angle origin and very small vessel diameters may prevent microcatheterization, and the bleeding site may only be accessible by the wire. The reperfusion catheter will allow temporary perfusion of the distal organ and mapping angiograms while access to the bleeding site is obtained.

Typically, there is no piece of hardware or embolic agent left behind by the embolization system, unless the probe tip is desired to be detached and left behind. In addition, there is no image artifacts in future control post embolization studies, such as in ultrasound, magnetic resonance, and computer tomography. Also there is no risk of non-target embolization.

## Claims

1. An energy-induced embolization system, comprising:
an introducer sheath having an inner wall defining a longitudinal opening extending from a proximal portion to a distal portion of the introducer sheath;
a first catheter having an outer wall and having a longitudinal opening extending from a proximal portion to a distal portion of the first catheter, the first catheter disposed through the longitudinal opening of the introducer sheath;
a conduit having a first end disposed in fluid communication with an inside portion of the introducer sheath and having a second end disposed in fluid communication with an inside portion of the first catheter, wherein antegrade blood flow is establishable in a lumen of a target vessel through a path between the inside portion of the introducer sheath, through an inside portion of the conduit, through the inside portion of the first catheter, and exiting through the distal portion of the first catheter; and
an energy delivery wire.

2. The system of claim 1, wherein the first end of the conduit is disposed in fluid communication with an area inside the introducer sheath between the inner wall of the introducer sheath and the outer wall of the first catheter.

3. The system of any preceding claim, further comprising a second catheter disposed through the longitudinal opening of the first catheter, preferably wherein the second catheter is a balloon catheter and preferably a balloon microcatheter, preferably, wherein the first catheter is not a balloon catheter.

4. The system of any preceding claim, further comprising a valve system disposed between the conduit and the first catheter.

5. The system of any preceding claim, further comprising a side port integrally formed in the introducer sheath and preferably further comprising a stopcock on the side port.

6. The system of any preceding claim, further comprising a tip at a distal end of the first catheter, the tip having an outer diameter smaller than the greatest outer diameter of the first catheter.

7. The system of any preceding claim, wherein the first catheter is a balloon catheter.

8. The system of any preceding claim, wherein the energy delivery wire is receivable through the longitudinal opening of the first catheter.

9. The system of any preceding claim, also comprising an access port for introduction of fluid into the inside portion of the conduit, preferably wherein the access port is selectively openable and closable and preferably wherein the access port is provided by a three-way stopcock in fluid communication with the inside portion of the conduit.

10. The system of any preceding claim, wherein the energy delivery wire includes a detachable probe.

11. The system of any preceding claim, wherein the energy delivery wire has a straight tip, helical tip, or V-shaped tip.

12. The system of any preceding claim, also comprising a control valve configured to allow or stop said antegrade blood flow, preferably wherein the control valve is in fluid communication with the inside portion of the conduit.

13. The system of any preceding claim, also comprising an energy delivery system operably associated with the energy delivery wire to deliver energy to the energy delivery wire, preferably wherein the energy delivery system is operable to deliver alternating electrical current to the energy delivery wire, preferably wherein the energy delivery system is operable to deliver radiofrequency energy to the energy delivery wire, preferably wherein the energy delivery system is a monopolar or bipolar energy delivery system.

14. The system of any preceding claim, wherein said inside portion of the introducer sheath is said longitudinal opening of the introducer sheath.

15. The system of any preceding claim, wherein said inside portion of the first catheter is said longitudinal opening of the first catheter.
